# EUROPEAN PATENT SPECIFICATION

(11) **EP 1 605 847 B1**
(45) Date of publication and mention of the grant of the patent: **16.09.2009**
(21) Application number: 04718503.8
(22) Date of filing: 08.03.2004
(51) Int. Cl.: A61K 41/00, A61K 47/48, A61P 27/02

(54) **ANTIBODY-TARGETED PHOTODYNAMIC THERAPY**
GEGEN ANTIKÖRPER GERICHTETE PHOTODYNAMISCHE THERAPIE
THERAPIE PHOTODYNAMIQUE CIBLEE SUR LES ANTICORPS

(30) Priority: 07.03.2003 US 452655 P
(43) Date of publication of application: 21.12.2005
(73) Proprietor: BOARD OF REGENTS, THE UNIVERSITY OF TEXAS SYSTEM, Austin, TX 78701 (US)
(72) Inventor: GLICKMAN, Randolph, D., San Antonio, TX 78240 (US); MAYO, George, L., Philadelphia, PA 19104 (US); MCKINNON, Stuart, J., San Antonio, TX 78248 (US); MELENDEZ, Robert, F., San Antonio, TX 78240 (US); KUMAR, Neeru, C., San Antonio, TX 78240 (US)
(74) Representative: Bradley, Adrian
(86) International application number: PCT/US2004/006985
(87) International publication number: WO 2004/080284

(56) References cited:
- WO-A-01/58240
- WO-A-02/07819
- WO-A-02/100326
- WO-A-03/039404
- US-B1- 6 319 273
- US-B1- 6 342 219
- US-B1- 6 443 976
- ALGVERE P V ET AL: "Age-related maculopathy: Pathogenetic features and new treatment modalities" ACTA OPHTHALMOLOGICA SCANDINAVICA 2002 UNITED KINGDOM, vol. 80, no. 2, 2002, pages 136-143, XP002465037 ISSN: 1395-3907
- MAYO G L ET AL: "Antibody-targeted photodynamic therapy" AMERICAN JOURNAL OF OPHTHALMOLOGY 2003 UNITED STATES, vol. 136, no. 6, 2003, pages 1151-1152, XP002465038 ISSN: 0002-9394
- DATABASE BIOSIS [Online] BIOSCIENCES INFORMATION SERVICE, PHILADELPHIA, PA, US; 2002, RENNO R ET AL: "Selective Targeting of Verteporfin to Choroidal Neovascularization Mediated by a Homing Peptide to VEGF-R2." XP002465048 Database accession no. PREV200300155549 & ARVO ANNUAL MEETING ABSTRACT SEARCH AND PROGRAM PLANNER, vol. 2002, 2002, page Abstract No. 3977, ANNUAL MEETING OF THE ASSOCIATION FOR RESEARCH IN VISION AND OPHTHALMOLOGY; FORT LAUDERDALE, FLORIDA, USA; MAY 05-10, 2002

## Description

### BACKGROUND

Photodynamic therapy is a medical procedure used for the treatment of a large variety of disease states, such as eye disorders, cancer, renal disorders, and skin disorders, among others. Photodynamic therapy putatively relies on a particularly active form of molecular oxygen known as singlet oxygen. Singlet oxygen, which is generally toxic to cells, can be generated in various ways. In photodynamic therapy, singlet oxygen is typically generated photochemically by irradiating a secondary substance known as a "photosensitizer" in the presence of air (oxygen).

As a method to treat cancer, photodynamic therapy attempts to induce malignant cell death and tumor elimination through a photochemical process triggered by irradiating diseased tissue with high-intensity light. Prior to irradiation, the diseased tissue is loaded with a photosensitizer that absorbs the light and uses part of its energy to drive a series of photochemical reactions. These photochemical reactions are believed to generate reactive toxic products such as singlet oxygen, which ultimately damage or destroy the diseased tissue.

A wide variety of photosensitizers have been used for photodynamic therapy, and the success of the therapy can depend in part on the particular photosensitizer utilized. For example, photosensitizers that strongly absorb on the edge of the visible region and into the near-infrared region are of special relevance since the penetration of light into living tissue is substantially better at wavelengths beyond 600 nm. The success of photodynamic therapy also rests largely on the preferential accumulation of photosensitizers by diseased tissue as compared to the adjacent healthy tissue, thereby allowing the destruction of such diseased tissue without damaging the surrounding healthy tissue. The limitations of photodynamic therapy are most often caused by poor performance of the photosensitizers in particular environments, or because the uptake and retention of a photosensitizer by a distinct diseased tissue is not sufficiently greater than that observed for the surrounding normal tissue.

One photosensitizer that has been used in photodynamic therapy is verteporfin. Verteporfin is sold in an injectable form under the name VISUDYNE (Parkedale Pharmaceuticals; Rochester, MN). Verteporfin has been used to treat choroidal neovascular disease (CNV) and has proved effective at preventing moderate to severe visual loss in eyes with subfoveal predominantly classic CNV or occult-only CNV caused by age-related macular degeneration (AMD) and in eyes with subfoveal CNV caused by pathologic myopia. (Lim, Ophthalmol Clin North Am; 15 (4): 473-478, vii, Dec. 2002). Verteporfin has also been used in photodynamic therapy for the treatment of pigment epithelial detachment (PED).

WO 02/100326 discloses methods that employ a dual mechanism of action against tumour growth by combining photodynamic therapy with anti cancer regimes. The methods utilize either (1) purified photosensitizer conjugate (PIC) composition comprising at least one photosensitizer and at least one solubilising agent each independently bound to an antibody through a covalent link or (2) a PIC composition comprising at least one photosensitizer covalently linked to an antibody wherein the density of PIC on the antibody quenches photoactivation during circulation in the bloodstream; and (3) a PIC comprising at least on photosensitizer bound to a PEGylated antibody.

Photodynamic therapy with photosensitizers such as verteporfin usually involve a two-step process, beginning with administration of the photosensitizer, e. g., by intravenous injection. While circulating in the bloodstream, the photosensitizer attaches to molecules called lipoproteins. Because cells undergoing rapid proliferation (cell division and growth) require a greater amount of lipoproteins than non-dividing cells, the photosensitizer is delivered more quickly and in higher concentrations to these types of cells. Once the concentration of photosensitizer reaches appropriate levels in a cell or tissue of interest, it is activated with a pre-calculated dose of light at a particular wavelength. The light dosage typically used for photodynamic therapy is usually much less damaging than thermal or hot laser treatment used in laser photocoagulation, which can leave permanent blind spots. The activated photosensitizer subsequently causes the conversion of normal oxygen found in tissue to singlet oxygen. The singlet oxygen, in turn, causes cell death by disrupting normal cellular functions.

Although verteporfin accumulates preferentially in choroidal neovasculature, some non-specific accumulation within the retina occurs. Thus, retinal structures may be damaged during the application of the light treatment. Needed in the art is the ability to increase the selectivity of photosensitizers like verteporfin to the specific tissue to be irradiated.

### SUMMARY

In one aspect, the present invention provides the use of a conjugate of a benzoporphyrin and an anti-VEGF antibody in me manufacture of a medicament for the treatment of ocular disease, by irradiating a subject to be treated with the medicament with light.

In a second aspect, the present invention provides a photosensitizer-antibody conjugate, comprising VISUDYNE bound to an anti-VEGF polyclonal antibody.

Additional advantages will be set forth in part in the description which follows, and in part will be obvious from the description, or may be learned by practice of the aspects described below. The advantages described below will be realized and attained by means of the elements and combinations particularly pointed out in the appended claims. It is to be understood that both the foregoing general description and the following detailed description are exemplary and explanatory only.

### BRIEF DESCRIPTION OF DRAWINGS

The accompanying drawings, which are incorporated in and constitute a part of this specification, illustrate several aspects described below.
Figure 1 is a pair of fluorescence excitation-emission scans of native verteporfin (top) and the verteporfin-anti-VEGF-conjugate (bottom). The spectra were collected on a Jobin-Yvon SPEX FL-3 spectrofluorimeter.
Figure 2A is a photograph of MS-1 vascular endothelial cells growing in 24-well plastic culture plate. Figure 2B is a close-up photograph of an individual well in the culture plate during exposure to the 647 nm emission of a Krypton-ion continuous wave laser.

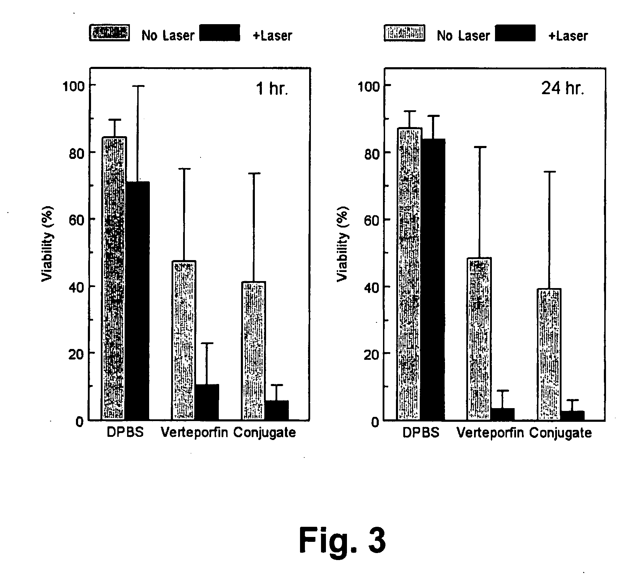

Figure 3 is a graph showing the mean percent cell viability by trypan blue exclusion over eight experimental runs in 24-well culture plates for VEGF-expressing endothelial cells in Dulbecco's phosphate buffered saline ("DPBS"), cells incubated with verteporfin (VISUDYNE) at 40 µg/ml ("Verteporfin"), and cells incubated with verteporfin (VISUDYNE) conjugated to anti-VEGF antibody ("Conjugate"). Error bars indicate 1 standard deviation from the mean. The cells received either no laser treatment or were exposed to krypton-ion CW laser (647 nm) at a total light dosage of 56.5 J/cm for either 1 hour (left) or 24 hours (right).
Figure 4A is an image of a confocal bright field view of MS-1 vascular endothelial cells growing on a glass coverslip. These cells were labeled with a VISUDYNE-anti-VEGF antibody conjugate. Figure 4B is an image of the same field as in "A," taken with fluorescence optics to image the presence of the conjugate. Images were made with an Olympus IX70 confocal fluorescence microscope (Olympus America, Inc.; Melville, NY). Fluorescence was excited with the 633 nm line of a HeNe laser, and the emission was acquired with a 660 nm longpass filter.
Figure 5A is a confocal fluorescence microscopic image of MS-1 vascular endothelial cells incubated with native VISUDYNE for 5 minutes. Figure 5B is a confocal fluorescence microscope image of MS-1 vascular endothelial cells incubated with the VISUDYNE-anti-VEGF antibody conjugate for 5 minutes.
Figure 6A is a confocal fluorescence microscope image of MS-1 vascular endothelial cells incubated with native VISUDYNE for 25 minutes. Figure 6B is a confocal fluorescence microscope image of MS-1 vascular endothelial cells incubated with VISUDYNE-anti-VEGF antibody conjugate for 20 minutes.
Figure 7 is a graph of fluorescence intensity of conjugate-labeled and VISUDYNE-labeled MS-1 vascular endothelial cells as a function of incubation time. The fluorescence intensity of confocal micrographs was determined with the "histogram" analysis tool of the ImagePro image processing software program (Media Cybernetics; Silver Springs, MD). Intensity is expressed as the accumulated pixel counts in the image.
Figure 8 is a graph of the photosensitizing properties, *i*.*e*., photoinduced cytotoxicity, of native verteporfin and the verteporfin-anti-VEGF antibody conjugate as a function of concentration.

### DETAILED DESCRIPTION

The disclosed materials, compositions, and uses may be understood more readily by reference to the following detailed description of specific aspects of the materials and methods and the Examples included therein and to the Figures and the previous and following description.

Before the present materials, compositions, and/or uses are disclosed and described, it is to be understood that the aspects described below are not limited to specific synthetic methods or specific reagents, as such may, of course, vary.

Disclosed are materials, compositions, and components that can be used for, can be used in conjunction with, can be used in preparation for, or are products of the disclosed use and compositions. These and other materials are disclosed herein, and it is understood when combinations, subsets, interactions, groups, etc. of these materials are disclosed that, while specific reference of each various individual and collective combinations and permutation of these compounds may not be explicitly disclosed, each is specifically contemplated and described herein. For example, if a conjugate is disclosed and discussed and a number of modifications that can be made to a number of photosensitizers and/or antibodies in the conjugate are discussed, each and every combination and permutation of the conjugate and the modifications to the photosensitizer and/or antibodies that are possible are specifically contemplated unless specifically indicated to the contrary. Thus, if a class of substituents A, B, and C are disclosed as well as a class of substituents D, E, and F and an example of a combination molecule, A-D is disclosed, then even if each is not individually recited, each is individually and collectively contemplated. Thus, in this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. This concept applies to all aspects of this disclosure including, steps in methods of making and using the disclosed compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

### Definitions

In this specification and in the claims which follow, reference will be made to a number of terms which shall be defined to have the following meanings:

As used in the specification and the appended claims, the singular forms "a," "an," and "the" include plural referents unless the context clearly dictates otherwise. Thus, for example, reference to "a conjugate" includes mixtures of one or more conjugates, reference to "an antibody" includes mixtures of one or more antibodies, reference to "the photosensitizer" includes one or more such photosensitizers, and the like.

Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

"Optional" or "optionally" means that the subsequently described event or circumstance may or may not occur, and that the description includes instances where the event or circumstance occurs and instances where it does not.

As used throughout, the term "subject" or "patient" can include domesticated animals (*e*.*g*., cat, dog, etc.), livestock (*e*.*g*., cattle, horse, pig, sheep, goat, etc.), laboratory animals (e.g., mouse, rabbit, rat, guinea pig, etc.) and birds. "Subject" or "patient" can also include a mammal, such as a primate or a human.

Reference herein to a "cell" or "tissue" can include a cell or tissue *in vitro.* Alternatively, reference to a "cell" or "tissue" can include a cell or tissue *in vivo*, which can be found in a subject. A "cell" or "tissue" can be a cell or tissue from any organism including, but not limited to, a bacterium, a eukaryote, or an animal.

The terms "higher," "increases," "elevates," or "elevation" refer to increases above basal levels, e. g., as compared to a control. The terms "low," "lower", "reduces", or "reduction" refer to decreases below basal levels, e. g, as compared to a control. By "control" is meant either a subject lacking a disease or a subject in the absence of a particular variable such as a therapeutic. A subject in the absence of a therapeutic can be the same subject before or after treatment with a therapeutic or can be a different subject in the absence of the therapeutic. Comparison to a control can include a comparison to a known control level or value known in the art. Thus, basal levels are normal *in vivo* levels prior to, or in the absence of, addition of an agent or another small molecule or ligand.

"Disease", as used herein means an impairment of the normal state of a subject or one of its parts that interrupts or modifies the performance of the function and is a response to environmental factors (e. g., malnutrition, industrial hazards, climate, injury), to infective agents (e. g., bacteria, fungus, or viruses), to inherent defects of the organism (e.g., genetic anomalies), or to combinations of these factors.

### Conjugate

A compound for use selectively targeting cells for destruction or ablation may comprise administering to a subject a photosensitizer conjugated to an antibody. The antibody allows for the selective targeting of an organ, tissue, or protein. That is, the antibody facilitates the localization of the photosensitizer- antibody conjugate to cells and tissues for which the antibody is designed to target. When light is directed onto the tissue or cell containing the photosensitizer- antibody conjugate, the photosensitizer becomes activated and the tissue or cell is destroyed where the light has been directed. Such activation can be local, focused activation, or can be systemic by general activation.

### Photosensitizers

The conjugates disclosed herein contain one or more photosensitizers, which are compounds that absorb light energy as specified in the claims. For example, red light can be used. Blue light can also be used. In one example, ambient light can be used. The photosensitizer can absorb light from about 600 nm to about 800 nm. In one example, the conjugate contains a photosensitizer as specified in the claims that can absorb light from about 650 nm to about 700 nm. In some aspects, the photosensitizer can absorb light of about 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 698, 699, 700, 701, 702, 703, 704, 705, 706, 707, 708, 709, 710, 711, 712, 713, 714, 715, 716, 717, 718, 719, 720, 721, 722, 723, 724, 725, 726, 727, 728, 729, 730, 731, 732, 733, 734, 735, 736, 737, 738, 739, 740, 741, 742, 743, 744, 745, 746, 747, 748, 749, 750, 751, 752, 753, 754, 755, 756, 757, 758, 759, 760, 761, 762, 763, 764, 765, 766, 767, 768, 769, 770, 771, 772, 773, 774, 775, 776, 777, 778, 79, 780, 781, 782, 783, 784, 785, 786, 787, 788, 789, 790, 791, 792, 793, 794, 795, 796, 797, 798, 799, or 800 nm, where any of the stated values can form an upper and/or lower endpoint when appropriate.

In one aspect, the photosensitize is a benzoporphyrin derivative, such as a benzoporphrin mono acid derivative. In another aspect, the photosenstitizer can be verteporfin, which is a benzoporphyrin derivative, or the injectable form of verteporfin, VISUDYNE®, from Parkedale Pharmaceuticals, Rochester, MN, which incorporates preferentially into choroidal neovasculature.

### Antibodies

The conjugates disclosed herein also contain one or more antibodies. In one aspect, the antibodies are monoclonal or polyclonal antibodies to vascular endothelial growth factor (anti-VEGF).

The term "antibodies" is used herein in a broad sense and includes both polyclonal and monoclonal antibodies. In addition to intact immunoglobulin molecules, also included in the term "antibodies" are fragments of immunoglobulin molecules and multimers of immunoglobulin molecules (*e*.*g*., diabodies, triabodies, and bi-specific and tri-specific antibodies, as are known in the art; *see, e*.*g*., Hudson and Kortt, J. Immunol. Methods 231:177-189, 1999), fusion proteins containing an antibody or antibody fragment, which are produced using standard molecular biology techniques, single chain antibodies, and human or humanized versions of immunoglobulin molecules or fragments thereof. Any antibody as specified in the claims that specifically binds an antigen in a manner sufficient to deliver a photosensitizer to the desired location can be used in the methods disclosed herein.

Whenever possible, antibodies useful in the conjugates and uses disclosed herein can be purchased from commercial sources, such as Chemicon International (Temecula, CA). The antibodies of the disclosed conjugates and methods can also be generated using well-known methods. The skilled artisan will understand that either full-length antigens or fragments thereof can be used to generate the antibodies of the disclosed conjugates and methods. A polypeptide to be used for generating an antibody of the disclosed conjugates and methods can be partially or fully purified from a natural source, or can be produced using recombinant DNA techniques. For example, for antigens that are peptides or polypeptides, a cDNA encoding an antigen, or a fragment thereof, can be expressed in prokaryotic cells (e. g., bacteria) or eukaryotic cells (e. g., yeast, insect, or mammalian cells), after which the recombinant protein can be purified and used to generate a monoclonal or polyclonal antibody preparation that specifically binds the targeted antigen.

One of skill in the art will know how to choose an antigenic peptide for the generation of monoclonal or polyclonal antibodies that specifically bind the appropriate antigens. Antigenic peptides for use in generating the antibodies of the disclosed conjugates and methods are chosen from non-helical regions of the protein that are hydrophilic. The PredictProtein Server (http://www.emblheidelberg.de/predictprotein/subunitdef.html) or an analogous program can be used to select antigenic peptides to generate the antibodies of the disclosed conjugates and methods. In one example, a peptide of about fifteen amino acids can be chosen and a peptide-antibody package can be obtained from a commercial source such as AnaSpec, Inc. (San Jose, CA). One of skill in the art will know that the generation of two or more different sets of monoclonal or polyclonal antibodies maximizes the likelihood of obtaining an antibody with the specificity and affinity required for its intended use. The antibodies are tested for their desired activity by known methods (e.g., but not limited to, ELISA and/or immunocytochemistry). For additional guidance regarding the generation and testing of antibodies, see, e. g., Harlow and Lane, Antibodies: A Laboratory Manual, Cold Spring Harbor Laboratory Press, Cold Spring Harbor, NY, 1988.

### Monoclonal antibodies

The term "monoclonal antibody" as used herein refers to an antibody or antibody fragment obtained from a substantially homogeneous population of antibodies or antibody fragments, i. e., the individual antibodies within the population are identical except for possible naturally occurring mutations that can be present in a small subset of the antibody molecules. The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, as long as they exhibit the desired antagonistic activity (See, e. g., U.S. Patent No. 4,816,567 and Morrison et al., Proc. Natl. Acad. Sci. USA, 81:6851-6855, 1984).

Monoclonal antibodies useful in the conjugates and uses disclosed herein can be prepared using hybridoma methods, such as those described by Kohler and Milstein, Nature, 256:495,1975. In a hybridoma method, a mouse or other appropriate host animal is typically immunized with an immunizing agent to elicit lymphocytes that produce or are capable of producing antibodies that will specifically bind to the immunizing agent. Alternatively, the lymphocytes can be immunized *in vitro,* e.g., using an adapter antigen or an immunogenic fragment thereof.

The monoclonal antibodies can also be made by recombinant DNA methods, such as those described in U.S. Patent No. 4,816,567 (Cabilly et al.). DNA encoding the monoclonal antibodies of the disclosed conjugates can be readily isolated and sequenced using conventional procedures (e.g., by using oligonucleotide probes that are capable of binding specifically to genes encoding the heavy and light chains of murine antibodies). Libraries of antibodies or active antibody fragments can also be generated and screened using phage display techniques, e.g., as described in U.S. Patent No. 5,804,440 (Burton et al.) and U.S. Patent No. 6,096,441 (Barbas et al.). Recombinant antibodies, antibody fragments, and fusions and polymers thereof can be expressed *in vitro* or in prokaryotic cells (e. g., bacteria) or eukaryotic cells (e.g., yeast, insect, or mammalian cells) and further purified, as necessary, using well known methods (see, e.g, Sambrook et al. Molecular Cloning: a Laboratory Manual, 3d Edition, Cold Spring Harbor Laboratory Press (2001); and Ausubel et al., Current Protocols in Molecular Biology, John Wiley & Sons, New York, NY, 2001, which is updated quarterly).

*In vitro* methods are also suitable for preparing monovalent antibodies. Digestion of antibodies to produce fragments thereof, particularly, Fab fragments, can be accomplished using routine techniques known in the art. For instance, digestion can be performed using papain. Examples of papain digestion are described in WO 94/29348 published Dec. 22, 1994 and U.S. Patent No. 4,342,566. Papain digestion of antibodies typically produces two identical antigen binding fragments, called Fab fragments, each with a single antigen binding site, and a residual Fc fragment. Pepsin treatment yields a fragment that has two antigen combining sites and is still capable of cross-linking antigen.

Any antibody or antibody fragment useful in the conjugates and uses disclosed herein, whether attached to other sequences or not, can also include insertions, deletions, substitutions, or other selected modifications of particular regions or specific amino acids residues, provided the activity of the antibody or antibody fragment is not significantly altered or impaired compared to the non-modified antibody or antibody fragment. These modifications can provide for some additional property, e.g., to remove or add amino acids capable of disulfide bonding, to increase its bio-longevity, to alter its secretory characteristics, etc. In any case, the antibody or antibody fragment must possess a bioactive property, such as specific binding to its cognate antigen. Functional or active regions of the antibody or antibody fragment can be identified and/or improved by mutagenesis of a specific region of the protein, followed by expression and testing of the expressed polypeptide. For example, amino acid sequence variants of antibodies or antibody fragments can be generated and those that display equivalent or improved affinity for antigen can be identified using standard techniques and/or those described herein. Methods for generating amino acid sequence variants are readily apparent to a skilled practitioner in the art and can include site-specific mutagenesis or random mutagenesis (e.g., by PCR) of the nucleic acid encoding the antibody or antibody fragment (Zoller, M. J. Curr. Opin. Biotechnol. 3:348-354, 1992). Both naturally occurring and non-naturally occurring amino acids (e.g, artificially-derivatized amino acids) can be used to generate amino acid sequence variants of the antibodies and antibody fragments used in the disclosed conjugates.

As used herein, the term "antibody" or "antibodies" can also refer to a human antibody and/or a humanized antibody. Many non-human antibodies (e. g., those derived from mice, rats, or rabbits) are naturally antigenic in humans, and thus can give rise to undesirable immune responses when administered to humans. Therefore, the use of human or humanized antibodies in the methods disclosed herein serves to lessen the chance that an antibody administered to a human will evoke an undesirable immune response.

### Human antibodies

The human antibodies useful in the conjugates and uses disclosed herein can be prepared using any technique. Examples of techniques for human monoclonal antibody production include those described by Cole et al. (Monoclonal Antibodies and Cancer Therapy, Alan R. Liss, p. 77, 1985) and by Boerner et al. (J. Immunol., 147(1):86-95, 1991). Human antibodies (and fragments thereof) useful in the conjugates and uses disclosed herein can also be produced using phage display libraries (Hoogenboom et al., J. Mol. Biol., 227: 381, 1991; Marks et al., J. Mol. Biol., 222:581,1991; and C.F. Barbas, D.R. Burton, J. K. Scott, G. J. Silverman, Phage Display: A Laboratory Manual, Cold Spring Harbor Laboratory Press, Cold Spring Harbor, NY, 2001).

The human antibodies useful in the conjugates and uses disclosed herein can also be obtained from transgenic animals. For example, transgenic, mutant mice that are capable of producing a full repertoire of human antibodies, in response to immunization, have been described (see, e.g., Jakobovits et al., Proc. Natl. Acad. Sci. USA, 90:2551-255,1993; Jakobovits et al., Nature, 362:255-258, 1993; Bruggermann et al., Year in Immunol., 7:33,1993). Specifically, the homozygous deletion of the antibody heavy chain joining region (J(H)) gene in these chimeric and germ-line mutant mice results in complete inhibition of endogenous antibody production, and the successful transfer of the human germ- line antibody gene array into such germ-line mutant mice results in the production of human antibodies upon antigen challenge.

### Humanized antibodies

Antibody humanization techniques generally involve the use of recombinant DNA technology to manipulate the DNA sequence encoding one or more polypeptide chains of an antibody molecule. Accordingly, a humanized form of a non-human antibody (or a fragment thereof) is a chimeric antibody or antibody chain (or a fragment thereof, such as an Fv, Fab, Fab', or other antigen-binding portion of an antibody), which contains a portion of an antigen binding site from a non-human (donor) antibody integrated into the framework of a human (recipient) antibody.

To generate a humanized antibody, residues from one or more complementarity determining regions (CDRs) of a recipient (human) antibody molecule are replaced by residues from one or more CDRs of a donor (non-human) antibody molecule that is known to have desired antigen binding characteristics (e.g., a certain level of specificity and affinity for the target antigen). In some instances, Fv framework (FR) residues of the human antibody are replaced by corresponding non-human residues. Humanized antibodies can also contain residues which are found neither in the recipient antibody nor in the imported CDR or framework sequences. Generally, a humanized antibody has one or more amino acid residues introduced into it from a source which is non-human. In practice, humanized antibodies are typically human antibodies in which some CDR residues and possibly some FR residues are substituted by residues from analogous sites in rodent antibodies. Humanized antibodies generally contain at least a portion of an antibody constant region (Fc), typically that of a human antibody (Jones et al., Nature, 321:522-525, 1986; Reichmann et al., Nature, 332:323-327, 1988; and Presta, Curr. Opin. Struct. Biol., 2:593-596, 1992).

Methods for humanizing non-human antibodies are well known in the art. For example, humanized antibodies can be generated according to the methods of Winter and co-workers (Jones et al., Nature, 321:522-525, 1986; Riechmann et al., Nature, 332:323-327, 1988; and Verhoeyen et al., Science, 239:1534-1536, 1988), by substituting rodent CDRs or CDR sequences for the corresponding sequences of a human antibody. Methods that can be used to produce humanized antibodies are also described in U.S. Patent No. 4,816,567 (Cabilly et al.), U.S. Patent No. 5,565,332 (Hoogenboom et al.), U.S. Patent No. 5,721,367 (Kay et al.), U.S. Patent No. 5,837,243 (Deo et al.), U.S. Patent No. 5, 939,598 (Kucherlapati et al.), U.S. Patent No. 6,130,364 (Jakobovits et al.), and U.S. Patent No. 6,180,377 (Morgan et al.).

### Photosensitizer-antibody coupling

Disclosed are compositions containing conjugates made up of an antibody or an antibody fragment coupled to a photosensitizer. The conjugates can be readily synthesized using techniques generally known to those of skill in the art. The starting materials and reagents used in preparing these conjugates are either available from commercial suppliers such as Aldrich Chemical Co., (Milwaukee, Wis.), Acros Organics (Morris Plains, N.J.), Fisher Scientific (Pittsburgh, Pa.), or Sigma (St. Louis, Mo.) or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991); March's Advanced Organic Chemistry, (John Wiley and Sons, 4th Edition); and Larock's Comprehensive Organic Transformations (VCH Publishers Inc., 1989).

In one aspect, the photosensitizer is coupled or linked to the antibody via a reactive chemical group on the photosensitizer and/or antibody such that the coupling between the photosensitizer and the antibody results in a covalent bond between the two that is resistant to reducing agents. Reactive chemical groups include, *e*.*g*., sulfhydryl groups, amino groups, carboxyl groups, and imidazole groups. The reactive chemical group can be in the hinge region of the antibody. This location reduces or eliminates interference between the antibody/antigen interaction and the photosensitizer. In one aspect, the photosensitizer can be coupled to the antibody, *e*.*g*., via a maleimide group.

The coupling of the photosensitizer to the antibody can also involve an activating agent. Various activating agents that can be used for the coupling reaction include, but are not limited to, 1-ethyl-3-(3-dimethylaminopropyl) carbodiimide (EDC), dicyclohexylcarbodiimide (DCC), *N*,*N*'-diisopropylcarbodiimide (DIP), benzotriazol-1-yl-oxy-tris-(dimethylamino)phosphonium hexa-fluorophosphate (BOP), hydroxybenzotriazole (HOBt), and N-methylmorpholine (NMM), including mixtures thereof. The coupling reaction can be carried out in solvents such as *N-*methylpyrrolidone (NMP) or in DMF. In one aspect, conjugation can involve the use of a conjugation kit, such as the Imject Immunogen EDC conjugation kit from Pierce (Rockford, IL). In another aspect, EDC and NMP can be obtained as separate reagents and formulated into a reaction mixture.

The photosensitizer-antibody conjugates as specified in the claims in general are specific for an antigen that allows targeting of the conjugates to an abnormally proliferative cell. In one aspect, the photosensitizer-antibody conjugate as specified in the claims can be specific for cells that express vascular endothelial growth factor (VEGF) or a portion thereof.

The disclosed photosensitizer-antibody conjugates can also include one or more additional biomolecules. The additional biomolecules can be coupled in the same manner as the photosensitizer. The biomolecules in an antibody conjugate can be the same as the photosensitizer molecule or different. More specifically, the biomolecules can have the same structure or different structures. Also, the disclosed photosensitizer-antibody conjugates can be combined with pharmaceutically acceptable carrier, as is described in detail herein.

Disclosed herein is the use of a conjugate for the manufacture of a medicament for the treatment of ocular disease, said treatment comprising administering to the subject a photosensitizer-antibody conjugate as specified in the claims and irradiating the subject with light. In this way, the subject's disease can be efficaciously treated as compared to a control lacking the conjugate or lacking the irradiation or both. Efficacious treatment is evaluated by detecting a reduction in one or more symptoms of the disease. An overall decrease, slowing, inhibition, and/or arrest of disease progress (e.g., further neovascularization), is indicative of efficacious treatment.

In one aspect, the photosensitizer-antibody conjugate comprises a benzoporphyrin and an anti-VEGF antibody. In another aspect, the benzoporphyrin is verteporfin or VISUDYNE.

The photosensitizer-antibody conjugates disclosed herein can be administered by any suitable means, such as intraperitoneal or intramuscular injection, intravenous injection, orally, by ocular or intranasal administration, or topically. In one aspect, the photosensitizer-antibody conjugate is administered by intravenous injection.

The photosensitizer-antibody conjugates disclosed herein can be infused within a biocompatible fluid, such as a physiological saline solution, or can be applied topically to the exterior surface of a tumor, eye, or abnormal tissue.

The light can be in the form of a laser or in the form of a fiber optic source used to deliver light to the treatment site from a laser. For example, the laser light can be shone through the slit lamp of a microscope into the subject's eye. In another example, the subject stands in a whole body light box containing lights of an appropriate wavelength. Light irradiation can also be accomplished by inserting a light source into the subject's body, e.g., by catheter or endoscope. The selection of the appropriate light source and wavelength can be performed by one of skill in the art and will depend on such factors as the type of photosensitizer being used, the type of and location of the site to be treated, and the like.

### Diseases treatable by a photosensitizer-antibody conjugate

Inappropriate angiogenesis and neovascularization is a physiological component of many types of diseases. Vascular endothelial cells both produce and respond to vascular endothelial growth factor (VEGF), which stimulates their proliferation, thereby stimulating angiogenesis and neovascularization. The photosensitizer-anti-VEGF antibody conjugates can be used to treat any ocular disease or condition that involves proliferation of endothelial cells and/or inappropriate angiogenesis or neovascularization. Antibodies against VEGF conjugated to a photosensitizer such as verteporfin can be used to treat ocular diseases involving abnormal endothelial cell growth and angiogenesis.

These diseases or conditions may occur in any human or non-human animal, e. g., but not limited to, horses, cows, sheep, goats, birds (e. g., chickens, geese, ducks, parrots, parakeets), dogs, cats, ferrets, mice, rats, hamsters, and guinea pigs.

### Uses in opthalmology

Many diseases or abnormal conditions of the eye involve the inappropriate stimulation of angiogenesis, which can lead to blindness. For example, proliferative diabetic retinopathy, in which there is abnormal proliferation of blood vessels on the surface of the retina, at the optic nerve, or in front of the eye on the iris, is a major cause of visual loss in diabetic patients. When vessels grow in front of the eye on the iris (iris neovascularization), they can clog the fluid outflow channels and cause the pressure to become very high (neovascular glaucoma); this condition can also be treated by the conjugates and methods disclosed herein. Corneal neovascularization is yet another eye disease that can be treated using the methods disclosed herein. The normal cornea is avascular; however, corneal insults, such as irritation secondary to contact lens wear, can induce corneal neovascularization, which can threaten vision directly or indirectly (e.g., through secondary hemorrhage, scarring, or lipid deposition.)

Choroidal neovascularization (CNV) is yet another ocular disease that threatens vision if left untreated. CNV can be occult CNV, classic CNV (minimally or predominately), or combinations thereof. Other diseases involving subfoveal neovessels in the eye include, but are not limted to, diabetic retinopathy, choroidal hemangioma, polypoidal choroidal vasculopathy, multifocal choroiditis and panuveitis, rubella retinopathy, angioid streaks, ocular histoplamosis syndrome, Vogt-Koyanagi-Harada syndrome, idiopathic subfoveal CNV, CNV in pathologic myopia, type 2A idiopathic juxtafoveolar retinal telangiectasis, and mallatia leventinese. These diseases can be treated using the conjugates disclosed herein.

In general, any disease or condition that causes retinal hypoxia or inflammation, thereby inducing abnormal neovascularization, can be treated using the methods and conjugates disclosed herein. Such diseases and conditions include sickle cell anemia, inflammatory diseases (including inflammatory diseases of the retina), hereditary dystrophies of the retina, trauma to the eye, and retinopathy of prematurity (i.e., in premature newborns).

The photosensitizer-anti-VEGF antibody conjugates disclosed herein can readily be administered ophthamically to any patient with a disease or condition involving abnormal angiogenesis in the eye. Following administration of the photosensitizer-antibody conjugate, the treated regions are exposed to the appropriate wavelength of laser light using routine methods, as will be understood by one of ordinary skill in the art, thereby abating some or all of the inappropriately proliferating endothelial cells and treating the disease. An overall decrease in abnormal neovascularization, and/or a slowing, inhibition, or arrest of disease progress (e.g., further neovascularization), is indicative of an efficacious treatment.

In one example, VISUDYNE, an injectable form of verteporfin, is used as the photosensitizer and is coupled to an antibody against VEGF. By increasing the affinity of VISUDYNE for endothelial cells, collateral damage is reduced, and the specificity of the photodynamic treatment is increased. The amount of VISUDYNE administered can be reduced to avoid or reduce undesired side-effects.

### Preventative uses

It is also contemplated that photosensitizer-antibody conjugates can be employed prophylactically to prevent the development of abnormal tissue changes at a prospective treatment site as specified in the claims.

### Residual tissue

Another application of the disclosed conjugates and uses is for destroying any residual tissue that remains after cataract surgery. For example, a photosensitizer conjugated to an antibody against lens epithelial cells can be used following cataract surgery to ablate residual lens epithelial cells that are thought to be the cause of posterior capsular opacification (PCO) that is a complication in up to 15% of cataract cases.

### Administration

The disclosed photosensitizer-antibody conjugates as specified in the claims can be conveniently formulated into pharmaceutical compositions composed of one or more of the conjugates in association with a pharmaceutically acceptable carrier. By "pharmaceutically acceptable carrier" is meant a material that is not biologically or otherwise undesirable. Thus, the carrier can be administered to a subject without causing undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art. See, e.g., Remington's Pharmaceutical Sciences, latest edition, by E.W. Martin Mack Pub. Co., Easton, PA, which discloses typical carriers and conventional methods of preparing pharmaceutical compositions that can be used in conjunction with the preparation of formulations of the conjugates disclosed herein.

Pharmaceutical formulations for the disclosed conjugates can include those suitable for parenteral (e.g., subcutaneous, intradermal, intramuscular, intravenous, intraperitoneal, and intraarticular) administration. Alternatively, pharmaceutical formulations of the disclosed conjugates can be suitable for administration to the mucous membranes of a subject (e.g., intranasal or ocular administration). The formulations can be conveniently prepared in unit dosage form and can be prepared by any of the methods well known in the art.

Depending on the intended mode of administration, the pharmaceutical compositions can be in the form of, for example, solids, semi-solids, liquids, solutions, suspensions (e.g., incorporated into microparticles, liposomes, etc.), emulsions, gels, or the like, preferably in unit dosage form suitable for single administration of a precise dosage. The pharmaceutical compositions can include, as noted above, an effective amount of the conjugate in combination with a pharmaceutically acceptable carrier and, in addition, can include other carriers, adjuvants, diluents, thickeners, buffers, preservatives, surfactants. Pharmaceutical compositions can also include one or more active ingredients such as other medicinal agents, pharmaceutical agents, antimicrobial agents, antiinflammatory agents, anesthetics.

Liquid pharmaceutically administrable compositions can, for example, be prepared by dissolving, dispersing, etc., a conjugate as described herein and optional pharmaceutical adjuvants in an excipient, such as, for example, water, saline aqueous dextrose, glycerol, ethanol, and the like, to thereby form a solution or suspension. If desired, the pharmaceutical composition to be administered can also contain minor amounts of nontoxic auxiliary substances such as wetting or emulsifying agents, pH buffering agents and the like, for example, sodium acetate, sorbitan monolaurate, triethanolamine sodium acetate, triethanolamine oleate, etc. Actual methods of preparing such dosage forms are known, or will be apparent, to those skilled in this art; for example see Remington's Pharmaceutical Sciences, referenced above.

In one aspect, when liposomes are used to deliver the conjugates disclosed herein, the liposomes can be lysed thermolitically by using, for example, a laser of the appropriate wavelength. Further, the liposome can be formulated to include agents such as indocyanine green to enhance the infrared laser absorption and improve the thermolysis of the liposomes. In this aspect, a laser, *e*.*g*., a laser of 810 nm wavelength, can be used to thermolitically lyse the liposomes. Such liposome thermolysis can be used to achieve local release of the conjugate near the target tissue. This can further reduce non-specific binding of the conjugate to non-targeted tissue.

Preparations for parenteral administration can include sterile aqueous or non-aqueous solutions, suspensions, and emulsions which may also contain buffers, diluents and other suitable additives. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles can include fluid and nutrient replenishers, electrolyte replenishers (such as those based on Ringer's dextrose), and the like. Preservatives and other additives may also be present such as, for example, antimicrobials, antioxidants, chelating agents, and inert gases and the like.

The dosage of the compositions required will vary from subject to subject, depending on the species, age, weight, the general condition of the subject, the severity of the disorder being treated, the particular active agent used, its mode of administration, physician judgment, and the like. Thus, it is not possible to specify an exact amount for every composition. However, an appropriate amount can be determined by one of ordinary skill in the art using only routine experimentation given the teachings herein.

In one aspect, the photosensitizer-antibody conjugates described herein are administered in an effective amount. The term "effective amount" is defined as any amount necessary to produce a desired physiologic response. Effective amounts and schedules for administering the compositions can be determined empirically, and making such determinations is within the skill in the art. The dosage ranges for the administration of the compositions are those large enough to produce the desired effect in which the symptoms or disorder are affected. The dosage should not be so large as to cause adverse side effects, such as unwanted cross-reactions, anaphylactic reactions, and the like. Generally, the dosage will vary with the age, condition, sex and extent of the disease in the patient, route of administration, or whether other drugs are included in the regimen, and can be determined by one of skill in the art. The dosage can be adjusted by the individual physician in the event of any contraindications. Dosage can vary, and can be administered in one or more dose administrations daily, for one or several days. Guidance can be found in the literature for appropriate dosages for given classes of pharmaceutical products.

In one example, the patient receives an intravenous injection of photosensitizer-antibody conjugate, and then waits 1 to 48 hours. For example, the wait can be from about 1-8 hours, from about 1-12 hours, from about 12-24 hours, from about 24-36 hours, or from about 36-48 hours. In yet another example, the wait is from about 24-36 hours. During this waiting period, the photosensitizer-antibody conjugate accumulates in the disease tissue to be treated and is removed from healthy tissue. After the waiting period, the patient returns to the clinic and the diseased tissue is illuminated for a given amount of time. The illumination time can be from about 1 minute to 4 hours, from about 5 minutes to 2 hours, or from about 10 to 30 minutes. In one aspect, the application of light is aimed directly at the abnormal tissue. If the tissue is internal, the light can be directed to the tissue or organ with devices such as a bronchoscope into the lungs or a cystoscope into the bladder, or with a catheter (U.S. Patent No. 5,454,794 (Narcisco et al.).

### Injection of Antibody/Photosensitizer Conjugates

It is generally preferable to introduce the photosensitizer-antibody conjugates as specified in the claims as close possible to a treatment site, such as by introducing the photosensitizer-antibody conjugate directly into a tumor. Furthermore, the cells that are targeted for destruction cannot be localized, but instead, can be viruses, microorganisms or metastasized cancer cells, which are more broadly distributed throughout a subject's body. It is therefore contemplated that the photosensitizer-antibody conjugate can be injected into the patient's bloodstream to allow the patient's own circulatory system to deliver the photosensitizer-antibody conjugates to the targeted tissue.

For example, a syringe can be used to inject a fluid containing the photosensitizer-antibody conjugates, which target a particular cell, in suspension through the skin and into the bloodstream. A needle passes through the skin and through a blood vessel wall; fluid containing the photosensitizer-antibody conjugates is injected through a needle into the blood. The blood flow in the vessel carries the photosensitizer-antibody conjugates to one or more locations where the targeted cells or microbes are disposed. The antibody will bind preferentially to the selected site. Therefore, injury to normal tissue is minimized during administration of the light, particularly, if the light is administered from an external source and must pass through normal tissue to reach the tissue that has been targeted.

### Methods of Measuring Treatment Effectiveness

In ocular treatment, verteporfin therapy stems the growth of CNV and prevents leakage from abnormal blood vessels. In one aspect, improved vision is characterized by a 10%, 20%, 30%, 40%, 50%, 60%, 70%, 80%, 90%, or 100% improvement in sight over a three-month follow-up period or a reduction in the extent of neurovascularization. In another aspect, an overall decrease, slowing, inhibition, and/or arrest of disease progress (e.g., further neovascularization), is indicative of efficacious treatment.

Efficacious treatment can also be characterized by from about 30-70%, from about 40-80%, from about 50-90%, or from about 60-100% reduction in target cell viability at one hour past exposure, as compared to a control. Efficacious treatment can further be characterized by from about 40-80%, from about 50-90%, from about 60-100%, or from about 70-100% reduction in target cell viability at twenty-four hours past exposure, as compared to a control.

### EXAMPLE

The following examples are put forth so as to provide those of ordinary skill in the art with a complete disclosure and description of how the compounds, compositions, and use described and claimed herein are made and evaluated, and are intended to be purely exemplary of what the inventors regard as their invention. Efforts have been made to ensure accuracy with respect to numbers (e.g., amounts, temperature, etc.) but some errors and deviations should be accounted for. Unless indicated otherwise, temperature is in °C or is at ambient temperature, and pressure is at or near atmospheric. There are numerous variations and combinations of reaction conditions, *e*.*g*., component concentrations, desired solvents, solvent mixtures, temperatures, pressures and other reaction ranges and conditions that can be used to optimize the product purity and yield obtained from the described process. Only reasonable and routine experimentation will be required to optimize such process conditions.

### Example 1

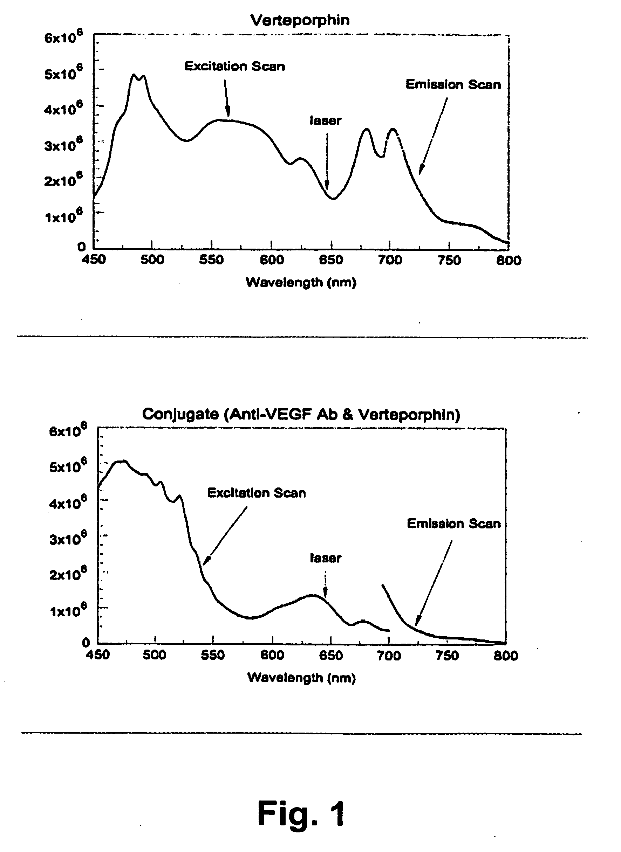

Rabbit anti-mouse VEGF polyclonal antibody was obtained from Chemicon International (Temecula, CA). The anti-VEGF antibody was conjugated to verteporfin (VISUDYNE ®; Parkedale Pharmaceuticals; Rochester, MN) using the 1-ethyl-3-[3-dimethylaminopropyl]-carbodiimide (EDC) crosslinking reagent obtained from Pierce (Rockford, IL), following the manufacturer's protocol. For each experimental run, 10 µg of anti-VEGF antibody were conjugated to 1 mg of verteporfin. The reaction product was purified by separation on a size exclusion column and eluted with phosphate buffered saline (PBS), thereby eliminating unconjugated verteporfin. The fluorescence excitation-emission spectrum was obtained from the conjugate (Fig. 1; bottom) and compared to that of verteporfin alone (Fig. 1; top).

The spectra shown in Fig. 1 were collected on a Jobin-Yvon SPEX FL-3 spectrofluorimeter. In the native verteporfin, the excitation peak at about 690 nm is typically used to produce the photodynamic action. The conjugate retains the fluorescence properties of verteporfin, although the relative amplitudes of the peaks are changed. The conjugate, however, retains a relative excitation peak at 690 nm, along with the corresponding emission peak at about 710 nm, similar to the fluorescence properties of native verteporfin. The arrow at 647 nm indicates the laser excitation derived from a Krypton-ion laser that was used in the example experiments. As is shown in the Fig. 1, the fluorescence excitation-emission spectrum of the conjugate (bottom) was found to be similar to that of verteporfin alone (top).

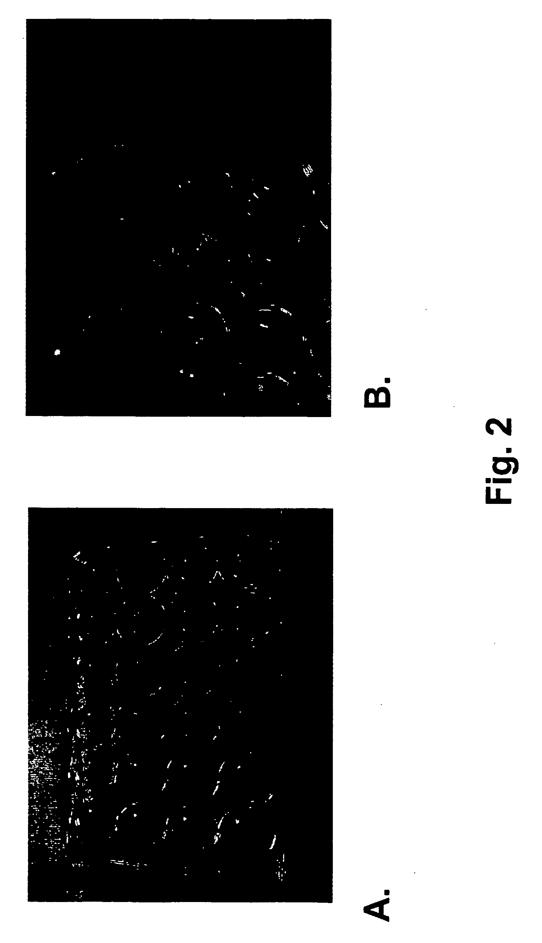

Experiments were conducted in a line of cultured, VEGF-expressing, murine endothelial cells (MS-1 VEGF, ATCC). Cells were grown in 24-well culture plates under standard cell culture conditions in Dulbecco's minimal essential medium (DMEM) with 10% fetal calf serum. Cells were incubated for one hour with either phosphate buffered saline solution alone, verteporfin at 40 µg/ml, or verteporfin-anti-VEGF antibody conjugate (Fig. 2A). All cells were washed twice with phosphate buffered saline following incubation to remove unbound photosensitizer. The photosensitizer was excited by exposing selected wells to the krypton-ion CW laser (647 nm) at a total light dosage of 56.5 J/cm, which is the recommended ophthalmic light dosage (Fig. 2A). For each treatment condition, there were laser-exposed and non-exposed groups. Following laser exposure, cells were incubated for either 1 or 24 hours, and cell viability was assessed using trypan blue exclusion.

The effect of photodynamic therapy was assessed based on changes in cell viability and the results are shown in Fig. 3. In the absence of laser exposure, there was no significant change in cell viability at 1 hour between conjugate and verteporfin treated cells. In both 1 hour and 24 hour laser-exposed groups, cells treated with either conjugate or verteporfin alone exhibited large losses of viability (P < 0.0001) compared with Dulbecco's PBS controls. Conjugated treated cells had uniformly lower viabilities than cells exposed to verteporfin; however, the difference did not reach statistical significance. Power calculations suggest that if the observed trend were to continue, statistical significance would be reached with 72 replicates. Cells exposed to verteporfin without laser exposure also showed reduced viability, indicating possible toxicity or low-level activation of the agent by ambient light.

### Example 2

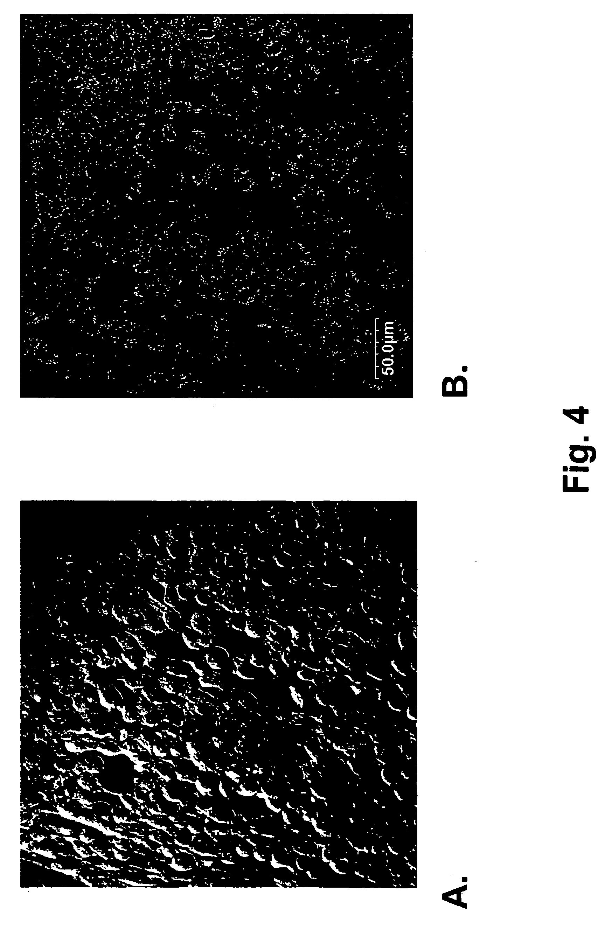

To demonstrate that VISUDYNE-anti-VEGF antibody conjugate is internalized into endothelial cell cytoplasm, a brightfield, confocal image of MS-1 VEGF-expressing vascular endothelial cells growing on a coverslip was taken. This image is shown in Figure 4A. A second image was taken of the same microscopic field of cells incubated with VISUDYNE-anti-VEGF antibody conjugate for 1 hour and then washed twice. This second image is shown in Figure 4B. The image shown in Figure 4B was taken with fluorescence optics to image the presence of the photosensitizer-antibody conjugate. The light areas represent the fluorescence due to VISUDYNE. Images were made with an Olympus IX70 confocal fluorescence microscope (Olympus America, Inc.; Melville, NY). Fluorescence was excited with the 633 nm line of a HeNe laser, and the emission was acquired with a 660 nm longpass filter.

### Example 3

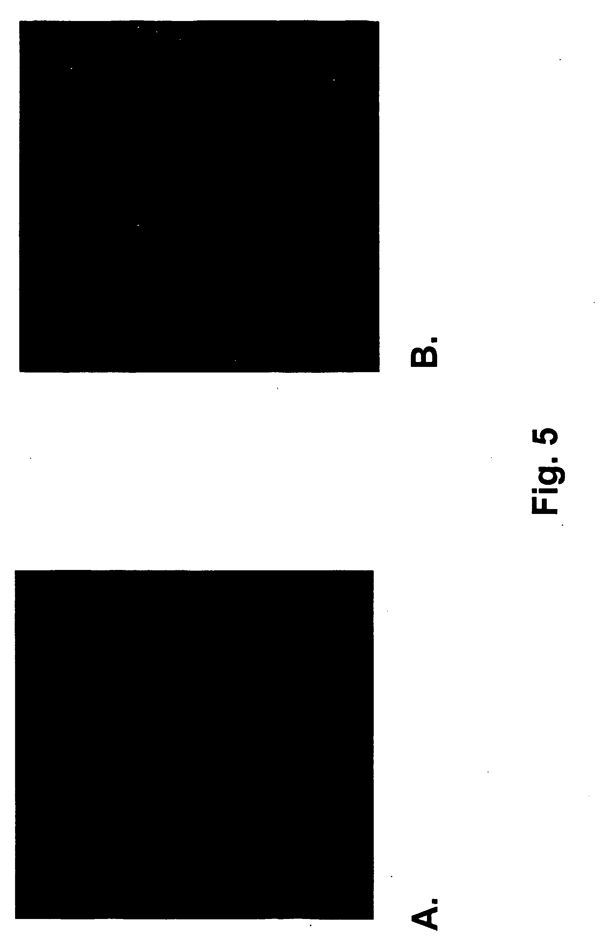

To demonstrate that VISUDYNE-anti-VEGF antibody conjugate is internalized more quickly than is native VISUDYNE, a confocal fluorescence microscopic image of MS-1 VEGF-expressing vascular endothelial cells cultured on a cover slip and incubated with native VISUDYNE for 5 minutes was taken. This image is shown in Figure 5A. All light areas represent VISUDYNE fluorescence. Comparisons were made of Figure 5A with the cells in the micrograph shown in Figure 5B, which were incubated for the same time period with VISUDYNE-anti-VEGF antibody conjugate. Surprisingly, as is evident from the two images, the VISUDYNE-anti-VEGF antibody conjugate penetrated into the endothelial cells more quickly than VISUDYNE alone.

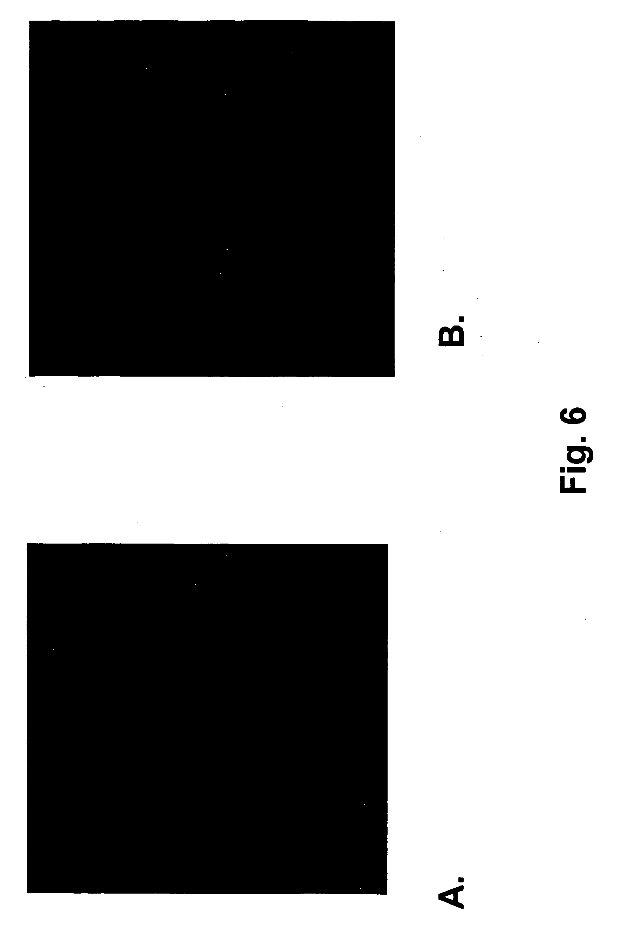

The images shown in Figure 6 were from the same conditions as those in Figure 5 except that the incubation time was 25 minutes in Figure 6A with native VISUDYNE and 20 minutes in Figure 6B with VISUDYNE-anti-VEGF antibody conjugate. Again, it is evident from the two images in Figure 6 that the VISUDYNE-anti-VEGF antibody conjugate penetrated into the endothelial cells more quickly than VISUDYNE alone, even though the incubation time with native VISUDYNE was longer.

### Example 4

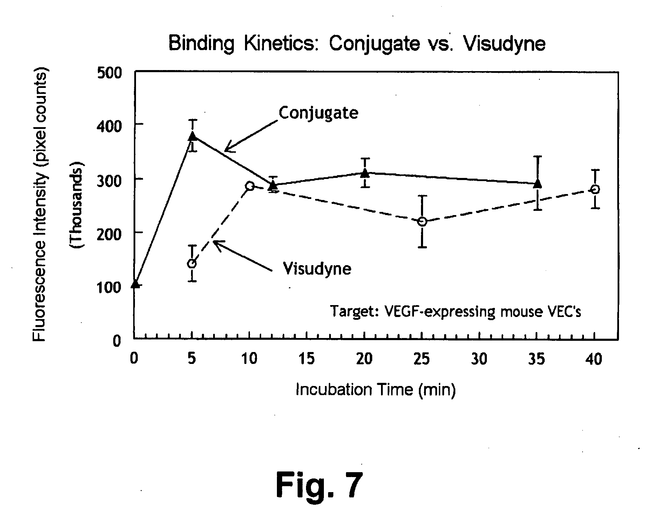

In order to provide a more quantitative comparison of the binding kinetics of the conjugate and the native VISUDYNE, the fluorescence intensity of the MS-1 cells labeled for varying durations was measured in a series of confocal micrographs using the "histogram" tool of ImagePro image processing program (Media Cybernetics; Silver Springs, MD). The results of such measurements are shown in Figure 7. It can be observed that within the first 10 minutes of labeling, the conjugate achieves effectively maximum binding to the target cells, whereas the native VISUDYNE reaches approximately 50% of maximum binding. Accordingly, if the laser activation were to be applied within 10 minutes of application of the conjugate, essentially maximum photodynamic effect would be realized. Targets not expressing the VEGF factor would not be as strongly labeled. This improved selectivity of the conjugate for VEGF-expressing targets is a distinct advantage over the native VISUDYNE.

### Example 5

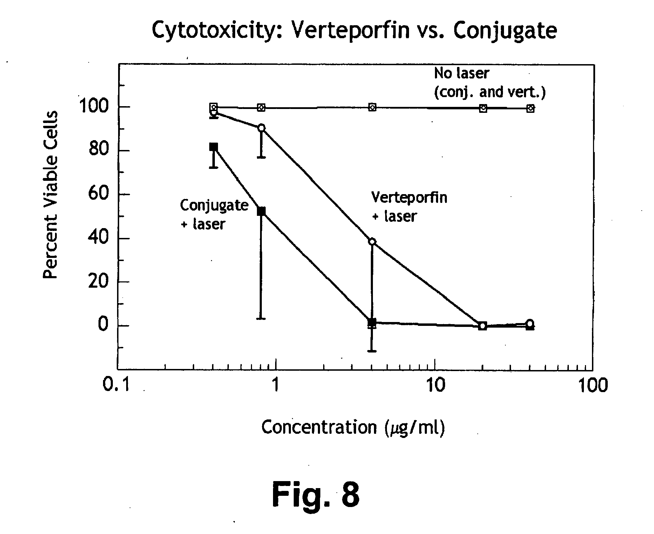

The relative efficiencies of native VISUDYNE and the VISUDYNE-anti-VEGF antibody conjugate were examined with respect to their photosensitizing properties. For the viability experiments, the MS-1 VEGF-expressing cells were plated on coverslips and grown in 6-well culture plates. The agents to be tested were applied to the cells in the various wells on the plate, incubated for one hour, exposed to the 647 nm laser, and incubated for an additional hour following the laser. To detect dead and dying cells, the Sytox Orange nuclear stain was obtained from Molecular Probes (Eugene, OR) and used at a concentration of 0.25 µM and an incubation time of 10 minutes. Total cell counts (live and dead) were made by counterstaining with Hoechst 33342 (Molecular Probes; Eugene, OR) at 20 µM and a 2-minute incubation period. The coverslips containing the cells were removed from the culture plate, and fluorescent cells were imaged with an Olympus BX60 epifluoresence microscope (Olympus America, Inc.; Melville, NY). Images were captured with a frame grabber, and analyzed with ImagePro software (Media Cybernetics; Silver Springs, MD) to obtain total cell counts with each stain. Three fields were measured per experimental condition. A summary of these results is shown in Figure 8.

As can be seen in Figure 8, the verteporfin (VISUDYNE)-anti-VEGF antibody conjugate photosensitizes the MS-1 cells at a lower concentration than does the native verteporfin (VISUDYNE). The difference in effect is probably greater than indicated, because the precise concentration of the conjugate is not known. When the conjugate was made, the concentration of the reagents was adjusted so that the initial concentration of verteporfin. (VISUDYNE) was the same as in the native verteporfin (VISUDYNE) preparation (40 µg/ml), The efficiency of the reaction and the recovery efficiency after the purification on the size-exclusion column, however, have not been determined, but both were likely to have been less than 100 percent. Therefore, the effective concentration of the conjugate was probably much less than indicated in Figure 8.

## Claims

1. The use of a conjugate of a benzoporphyrin and an anti-VEGF antibody in the manufacture of a medicament for the treatment of ocular disease, by irradiating a subject to be treated with the medicament with light.

2. The use of claim 1, wherein the benzophorphyrin is verteporfin.

3. The use of claim 2, wherein the verteporfin is VISUDYNE.

4. The use of any preceding claim, wherein the anti-VEGF antibody is a polyclonal antibody.

5. The use of any of claims 1 to 3, wherein the anti-VEGF antibody is a monoclonal antibody.

6. The use of any preceding claim, wherein the ocular disease is **characterized by** neovascularization.

7. The use of any preceding claim, wherein the ocular disease is iris neovascularization.

8. The use of any of claims 1 to 6, wherein the ocular disease is corneal neovascularization.

9. The use of any of claims 1 to 6, wherein the ocular disease is choroidal neovascularization.

10. The use of claim 9, wherein the choroidal neovascularization is occult-choroidal neovascularization.

11. The use of claim 9, wherein the choroidal neovascularization is classic choroidal neovascularization.

12. The use of any one of claims 1 to 6, wherein the ocular disease is proliferative diabetic retinopathy.

13. The use of any one of the preceding claims, wherein said medicament is for administration by intravenous injection of the conjugate.

14. The use of claim 1, wherein said medicament is for administration to a subject by contact with an ophthalmic solution of the conjugate.

15. The use of any preceding claim, wherein the light is from about 600 nm to about 800 nm.

16. A photosensitizer-antibody conjugate, comprising VISUDYNE bound to an anti-VEGF polyclonal antibody.

## Patentansprüche

1. Verwendung eines Konjugats eines Benzoporphyrins und eines Anti-VEGF-Antikörpers bei der Herstellung eines Medikaments für die Behandlung einer Augenkrankheit, durch Bestrahlen einer mit dem Medikament zu behandelnden Person mit Licht.

2. Verwendung nach Anspruch 1, wobei das Benzoporphyrin Verteporfin ist.

3. Verwendung nach Anspruch 2, wobei das Verteporfin VISUDYNE ist.

4. Verwendung nach einem beliebigen der vorhergehenden Ansprüche, wobei der Anti-VEGF-Antikörper ein polyklonaler Antikörper ist.

5. Verwendung nach einem beliebigen der Ansprüche 1 bis 3, wobei der Anti-VEGF-Antikörper ein monoklonaler Antikörper ist.

6. Verwendung nach einem beliebigen der vorhergehenden Ansprüche, wobei die Augenkrankheit durch Neovaskularisation **gekennzeichnet** ist.

7. Verwendung nach einem beliebigen der vorhergehenden Ansprüche, wobei die Augenkrankheit Neovaskularisation der Iris ist.

8. Verwendung nach einem beliebigen der Ansprüche 1 bis 6, wobei die Augenkrankheit Neovaskularisation der Hornhaut ist.

9. Verwendung nach einem beliebigen der Ansprüche 1 bis 6, wobei die Augenkrankheit choroidale Neovaskularisation ist.

10. Verwendung nach Anspruch 9, wobei die choroidale Neovaskularisation okkulte choroidale Neovaskularisation ist.

11. Verwendung nach Anspruch 9, wobei die choroidale Neovaskularisation klassische choroidale Neovaskularisation ist.

12. Verwendung nach einem beliebigen der Ansprüche 1 bis 6, wobei die Augenkrankheit proliferative diabetische Retinopathie ist.

13. Verwendung nach einem beliebigen der vorhergehenden Ansprüche, wobei das Medikament für die Verabreichung durch intravenöse Injektion des Konjugats gedacht ist.

14. Verwendung nach Anspruch 1, wobei das Medikament für die Verabreichung an eine Person durch Kontakt mit einer ophtalmischen Lösung des Konjugats gedacht ist.

15. Verwendung nach einem beliebigen der vorhergehenden Ansprüche, wobei das Licht zwischen ungefähr 600 nm bis ungefähr 800 nm liegt.

16. Photosensitizer-Antikörper-Konjugat, umfassend VISUDYNE, gebunden an einen polyklonalen Anti-VEGF-Antikörper.

## Revendications

1. Utilisation d'un conjugué d'une benzoporphyrine et d'un anticorps anti-VEGF dans la fabrication d'un médicament pour le traitement d'une maladie oculaire, en irradiant un sujet à traiter avec le médicament avec de la lumière.

2. Utilisation selon la revendication 1, dans laquelle la benzoporphyrine est de la vertéporfine.

3. Utilisation selon la revendication 2, dans laquelle la vertéporfine est VISUDYNE.

4. Utilisation selon l'une quelconque des revendications précédentes, dans laquelle l'anticorps anti-VEGF est un anticorps polyclonal.

5. Utilisation selon l'une quelconque des revendications 1 à 3, dans laquelle l'anticorps anti-VEGF est un anticorps monoclonal.

6. Utilisation selon l'une quelconque des revendications précédentes, dans laquelle la maladie oculaire est **caractérisée par** une néovascularisation.

7. Utilisation selon l'une quelconque des revendications précédentes, dans laquelle la maladie oculaire est une néovascularisation de l'iris.

8. Utilisation selon l'une quelconque des revendications 1 à 6, dans laquelle la maladie oculaire est une néovascularisation cornéenne.

9. Utilisation selon l'une quelconque des revendications 1 à 6, dans laquelle la maladie oculaire est une néovascularisation choroïdienne.

10. Utilisation selon la revendication 9, dans laquelle la néovascularisation choroïdienne est une néovascularisation occulte choroïdienne.

11. Utilisation selon la revendication 9, dans laquelle la néovascularisation choroïdienne est une néovascularisation choroïdienne classique.

12. Utilisation selon l'une quelconque des revendications 1 à 6, dans laquelle la maladie oculaire est une rétinopathie diabétique proliférante.

13. Utilisation selon l'une quelconque des revendications précédentes, dans laquelle ledit médicament est destiné à l'administration par injection intraveineuse du conjugué.

14. Utilisation selon la revendication 1, dans laquelle ledit médicament est destiné à l'administration à un sujet par contact avec une solution ophtalmique du conjugué.

15. Utilisation selon l'une quelconque des revendications précédentes, dans laquelle la lumière est d'environ 600 nm à environ 800 nm.

16. Conjugué photosensibilisateur - anticorps, comprenant VISUDYNE lié à un anticorps polyclonal anti-VEGF.
